## Europäisches Patentamt
## European Patent Office
## Office européen des brevets

(11) Veröffentlichungsnummer : **0 133 471**
**B1**

(12) **EUROPÄISCHE PATENTSCHRIFT**

(45) Veröffentlichungstag der Patentschrift :
11.11.87

(51) Int. Cl.⁴ : **A 61 M 5/30**

(21) Anmeldenummer : **84107582.3**

(22) Anmeldetag : **29.06.84**

(54) **Nadelloses Impfgerät , z.B. zur Eingabe von Insulin.**

(30) Priorität : **29.06.83 HU 235083**

(43) Veröffentlichungstag der Anmeldung :
**27.02.85 Patentblatt 85/09**

(45) Bekanntmachung des Hinweises auf die Patenterteilung : **11.11.87 Patentblatt 87/46**

(84) Benannte Vertragsstaaten :
**DE FR GB IT NL**

(56) Entgegenhaltungen :
**DE-A- 1 791 094**
**DE-A- 1 944 006**
**US-A- 2 645 223**
**US-A- 2 653 602**
**US-A- 3 292 622**
**US-A- 3 526 225**

(73) Patentinhaber : **RADELKIS Elektrokémiai Müszergyárto Szövetkezet**
**Laborc u. 1**
**· H-1300 Budapest (HU)**

(72) Erfinder : **Fejes, Kalman, Dipl.-Ing.**
**Vércse u. 9**
**H-1213 Budapest (HU)**
Erfinder : **Nagy, Lajos, Dipl.-Ing.**
**Fodor u. 5-7**
**H-1126 Budapest (HU)**

(74) Vertreter : **Patentanwälte Viering & Jentschura**
**Steinsdorfstrasse 6**
**D-8000 München 22 (DE)**

**Beschreibung**

Im Laufe der Entwicklung der verschiedenen, zu therapeutischen Zwecken dienenden technischen Mittel wurden auch solche nadellosen Impfgeräte entwickelt, mit denen die in der Humanheilkunde hinreichende Vakzinemenge von 1,5 cm³ durch einmaliges Einspritzen in den Organismus eingeführt werden kann.

Von den Geräten solchen Typs sollen das in der UdSSR hergestellte Impfgerät « Bienchen », das in USA entwickelte « Jet » und das ungarische patentierte Impfgerät, das unter dem Namen « Viper » bekannt ist, erwähnt werden. Die oben erwähnten Geräte erhalten die zum Ausschießen der Vakzine erforderliche Energie auf verschiedene Weisen unter Zwischenschaltung eines Druckerzeugers. Demnach wird die Voraussetzung der Betätigung durch die Zwischenschaltung einer Hydraulikpumpe, mittels welcher eine den Impfkolben antreibende Feder vorgespannt wird (US-A-3 526 225), oder durch eine kombinierte, aus einem Hebe- und Auszieharm mit Hebespindel bestehende Konstruktion sichergestellt. Die Nachteile der erwähnten Geräte sind die folgenden : durch die komplizierte Ausführung sind für die Bedienung und Wartung unbedingt Fachkenntnisse erforderlich, infolge der großen Dimensionen und des Gewichtes sind Spezialwerkzeuge unerläßlich, und außerdem sind die Produktionskosten beträchtlich. Wegen der erwähnten Nachteile konnten die bekannten Impfgeräte auf dem Markt nicht den gewünschten Absatz erzielen, da sie u. a. für Kranke, die ständig Insulin beanspruchen, zu kompliziert und zu aufwendig sind.

Es ist auch ein nadelloses Impfgerät mit den Merkmalen aus dem einleitenden Teil des Patentanspruches bekannt (US-A-3 292 622). Dadurch, daß der Impfkolben über den Arbeitskolben unmittelbar von dem plötzlich zugeführten Druckgas beschleunigt wird, wird eine sichere Funktion des Impfgeräts erreicht. Wenn bei dem bekannten Impfgerät der Vorwärtshub des Impfkolbens zur Änderung der auszugebenden Impfstoffmenge geändert werden soll, wird der Eichkörper, der zu der Hubbegrenzung des Impfkolbens mit dem gehäusefesten Endanschlag zusammenwirkt, gegen einen Eichkörper mit entsprechend anderer Länge ausgetauscht. Hierdurch kann auf eine die relative axiale Position des Impfkolbens einstellende Schraubkonstruktion verzichtet werden, die bei anderen bekannten Impfgeräten zur Einstellung der gewünschten Vakzinemenge diente. Demgegenüber kann mithilfe auswechselbarer Eichkörper die Vakzinemenge mit höchster Genauigkeit eingestellt werden, so daß die sich aus einer Verstellung der erwähnten Schraubkonstruktion ergebenden Probleme vollkommen eliminiert sind.

Bei dem bekannten Impfgerät ist die Kolbenstange des Impfkolbens mit einer hohlen Kolbenstange des Arbeitskolbens, die aus der Rückseite der Betätigungseinheit herausgeführt ist, mittels einer durch die Kolbenstange des Arbeitskolbens hindurchgeführten Schraubstange verschraubt, von welcher der Eichkörper mit dem herausragenden Ende der Kolbenstange des Arbeitskolbens verspannt wird. Die Gehäuserückseite der Betätigungseinheit wirkt als gehäusefester Endanschlag unmittelbar mit dem Eichkörper zusammen. Dadurch wird jedoch die Schraubverbindung zwischen dem Eichkörper und der Kolbenstange bei jedem Auftreffen desselben auf die Gehäuserückseite mit einem verhältnismäßig starken Stoß belastet. Falls sich durch die Stöße bei wiederholter Betätigung die Schraubverbindung lockert, werden der Vorwärtshub des Impfkolbens und daher die ausgegebene Impfstoffmenge unabsichtlich verstellt.

Durch die Erfindung gemäß dem Patentanspruch wird bei einfacher und zuverlässiger Konstruktion des Impfgerätes erreicht, daß die Verbindung zwischen dem Eichkörper und der Kolbenstange des Impfkolbens von starken Stoßbeanspruchungen am Ende des Dosierhubes des Impfkolbens entlastet ist.

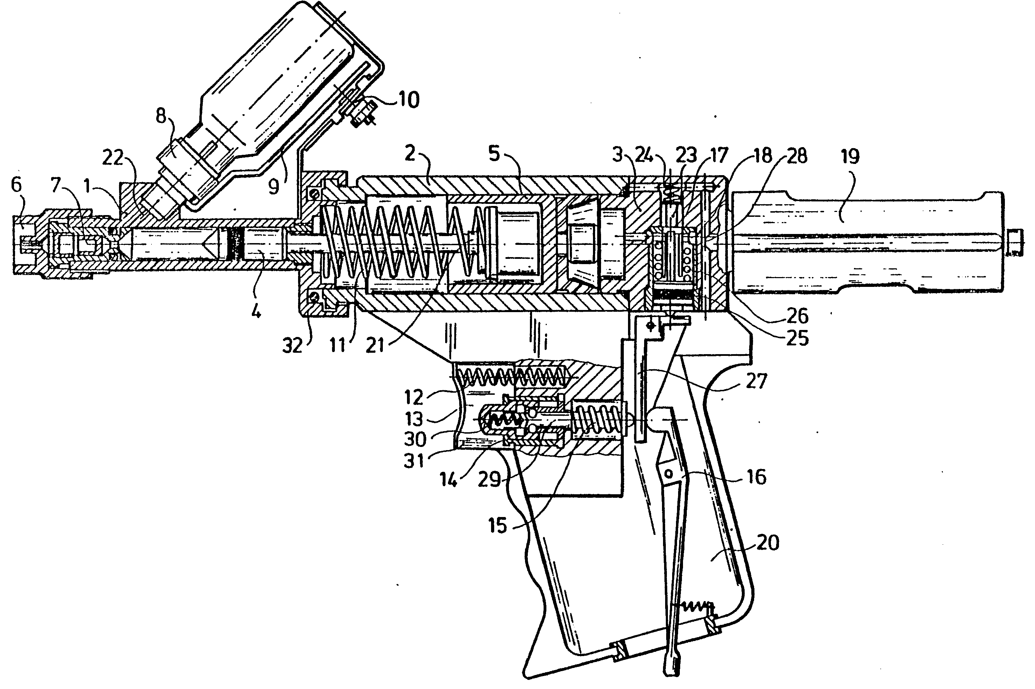

Die Erfindung wird im folgenden anhand einer aus der beiliegenden Zeichnung, in der das erfindungsgemäße Impfgerät im Längsschnitt dargestellt ist, ersichtlichen vorteilhaften Ausführungsform näher erläutert.

Wie aus der Figur ersichtlich, weist das erfindungsgemäße Impfgerät eine Impfeinheit und eine Betätigungseinheit 2 auf, die miteinander über eine manuell lösbare Schnellverbindung 32 verbunden sind, die beispielsweise als Bajonettverschluß oder als federbelasteter Druckverschluß ausgebildet sein kann.

In einem Impfzylinder 1 der Impfeinheit ist, um den für das Ausschießen des Impfstoffes erforderlichen Druck aufzubringen, ein Impfkolben 4 angeordnet. Wird nun der Impfkolben 4 in Richtung zur Betätigungseinheit 2 hin verschoben, so wird die Vakzine aus einer Vorratsampulle 8, 9, die an der Impfeinheit mittels des Bügels 10 befestigt ist, über eine Durchgangsbohrung 22 in den Impfzylinder 1 eingesaugt.

Diese Verschiebung des Impfkolbens 4 findet mit Hilfe einer als Schraubenfeder ausgebildeten Rückholfeder 11 statt.

An dem der Betätigungseinheit zugewandten Ende des Impfkolbens 4 ist ein zur Einstellung der Dosis dienender Eichkörper 21 vorgesehen. Wird der Impfkolben 4 nach rückwärts bewegt, so wird die Vakzine in den Impfzylinder 1 eingesaugt. Bewegt sich der Impfkolben 4 dann in die entgegengesetzte Richtung, also nach vorwärts, verläßt die Vakzine über ein Rückschlagventil 7 und einen Impfkopf 6 mit einer derartigen Energie das Impfgerät, daß eine nadellose Injektion der Vakzine möglich ist.

In dem Gehäuse der Betätigungseinheit 2 ist ein Arbeitskolben 5 angeordnet, der durch die aus einer Kohlensäurepatrone 19, die über ein Patroneneinzugsmittel befestigt ist, durch ein Druckregelventil 18 hindurchströmende Kohlensäure betätigt wird und dabei den Impfkolben 4 nach vorn stößt.

Im hinteren Teil der Betätigungseinheit 2 ist zwischen dem Arbeitskolben 5 und der Siphonpatrone 19 ein das Druckregelventil 18 steuernder Kolben 25 angeordnet, dessen Längsachse quer zu der des Arbeitskolbens 5 verläuft. Der Kolben 25 ist über einen balligen Betätigungsteil, der an dem dem Handgriff 20 zugewandten Ende des Kolbens 25 ausgebildet ist und mit dem kürzeren Arm eines Winkelhebels 27 in Eingriff steht, und gegen die Kraft einer Feder 28 verschiebbar. Wird nun der Winkelhebel 27 im Gegenuhrzeigersinn um seine Lagerachse verschwenkt, so wird von dem oberen, dem Handgriff 20 abgewandten Ende des Kolbens 25 ein an einer Feder 24 abgestützter Dichtungskörper 23 verschoben, wodurch der Durchtritt für das aus der Kohlensäurepatrone austretende Druckgas über ein im hinteren Teil des Gehäuses 3 ausgebildetes Kanalsystem zum Arbeitskolben 5 hin freigegeben wird.

Bei einer Schwenkbewegung des Winkelhebels 27 im Uhrzeigersinn gelangt der Kolben 25 unter der Wirkung der Feder 28 in seine durch einen Anschlag bestimmte Ausgangsstellung, wodurch der von dem Arbeitskolben 5 begrenzte Druckraum über eine Bohrung 26 des Kolbens 25 zur Umgebung hin entlüftet wird. Gleichzeitig wird von dem Dichtungskörper 23 ein weiteres Ausströmen der Kohlensäure aus der Patrone 19 verhindert.

Vor der Betätigung des Impfgerätes wird mit Hilfe eines Spannhebels 16 ein Auslösebolzen 29 gegen die Kraft einer Auslösefeder 15 so lange verschoben, bis drei Fixierkugeln 30 in eine im Bolzen 29 ausgebildete Rille einrasten. Die Fixierkugeln 30 legen den Auslösebolzen 29 in seiner vorderen Position fest.

Wenn die von der Rückstellfeder 12 belastete Auslösetaste 13 angezogen wird, wird eine das Austreten der Fixierkugeln 30 aus der Rille des Auslösebolzens 29 verhindernde Gleithülse 31 soweit verschoben, bis die Fixierkugeln 30 in eine Innennut der Gleithülse 31 eintreten können und dadurch den Auslösebolzen 29 freigeben. Der freigegebene Auslösebolzen 29 wird von der Auslösefeder 15 nach rückwärts bewegt, wodurch der Winkelhebel 27 im Gegenuhrzeigersinn verschwenkt wird, wodurch der Arbeitskolben 5 von dem Druckgas beaufschlagt wird und der Impfstoff von dem Impfkolben 4 ausgestoßen wird.

Bei der aus der Zeichnung ersichtlichen bevorzugten Ausführungsform ist der in der Betätigungseinheit verschiebbare Arbeitskolben 5 an seiner dem Impfkolben 4 zugewendeten Seite topfförmig ausgebildet, so daß er den Eichkörper 21 am hinteren Ende der Kolbenstange des Impfkolbens 4 umschließt und bei seiner Vorwärtsverschiebung mit seinem Topfboden an dem Eichkörper 21 anschlägt, wodurch der Impfkolben 4 nach vorn gestoßen wird. Der Bewegungshub des Arbeitskolbens 5 wird nach vorn durch eine mit dem Topfrand zusammenwirkende Anschlagschulter an der Betätigungseinheit 2 begrenzt, wohingegen die hintere Stellung des Impfkolbens 4 von einer in der Impfeinheit ausgebildeten Anschlagschulter begrenzt wird. Da die Ausgangsstellung des Arbeitskolbens 5 und daher dessen Abstand von der seine vordere Stellung begrenzenden Anschlagschulter somit von der Axialstellung des Eichkörpers 21 in der hinteren Stellung des Impfkolbens 4 bestimmt wird, wird auch der Arbeitshub des Arbeitskolbens 5 und somit des Impfkolbens 4 von der Axialstellung des Eichkörpers 21 bzw. dessen Abstand von dem Impfkolben 4 bestimmt. Durch Verändern dieses Abstandes nach dem Lösen der Schnellverbindung 32 kann daher die ausgegebene Impfstoffmenge genau eingestellt werden. Hierzu könnte der Eichkörper 21 auf der Kolbenstange des Impfkolbens auch schraubverstellbar sein, wobei eine Skala vorgesehen sein könnte, an welcher die Axialstellung des Eichkörpers 21 auf der Kolbenstange abgelesen werden kann.

## Patentanspruch

Nadelloses Impfgerät mit einer einen Impfzylinder (1) enthaltenden Impfeinheit und einer Betätigungseinheit (2), welche über eine manuell lösbare Schnellverbindung (32) miteinander verbunden sind, einem in dem Impfzylinder (1) gegen die Kraft einer Rückholfeder (11) durch Druckbeaufschlagung eines in der Betätigungseinheit (2) angeordneten Arbeitskolbens (5) mit dem Gasdruck aus einer Siphonpatrone (19) vorwärtsverschiebbaren Impfkolben (4), einer spannbaren Auslösevorrichtung (13, 15, 29, 30) zum plötzlichen Betätigen eines Ventils (18) zur Druckbeaufschlagung des Arbeitskolbens (5), einem an der Kolbenstange des Impfkolbens (4) befestigten Eichkörper (21) und einem an der Betätigungseinheit (2) vorgesehenen Endanschlag zur Begrenzung des Vorwärtshubes des Impfkolbens (4), dadurch gekennzeichnet, daß der Eichkörper (21) zwischen der druckbeaufschlagten Fläche des Arbeitskolbens (5) und dem Impfkolben (4) auf dem Ende von dessen Kolbenstange befestigt ist und der zur Verschiebung des Impfkolbens (4) an dem Eichkörper (21) angreifende Arbeitskolben (5) mit dem Endanschlag zusammenwirkt.

## Claim

An inoculating device without needle comprising an inoculating unit including an inoculating cylinder (1), an actuating unit (2) connected to the inoculating cylinder (1) by a quickly releasable coupling (32), an inoculating piston (4) being forwardly displacable in the inoculating cylinder (1) against the force of a return spring (11) by means of pressurized gas coming out of a siphon cartridge (19), the gas inpacting on a working piston (5) arranged in the actuating unit (2), a biasable actuating equipment (13, 15, 29, 30) for suddenly actuating a valve (18) resulting in the gas impact on the working piston (5), a calibrating element (21) fixed to the piston rod of the inoculating piston (2), and an end stop provided on the actuating unit (2) and limiting the forward stroke of the inoculating piston (4), characterized in that the calibrating element (21) is fixed on the end of the piston rod of the inoculating piston (4) between the gas impact surface of the working piston (5) and the inoculating piston (4), and the working piston (5), acting on the calibrating element (21) for displacing the inoculating piston (4) cooperates with the end stop.

## Revendication

Dispositif d'inoculation sans aiguille comportant une unité d'inoculation contenant un cylindre d'inoculation (1) et une unité d'actionnement (2), lesquelles sont accouplées par une fixation rapide (32) à déverrouillage manuel, un piston d'inoculation (4) apte à coulisser vers l'avant, grâce à la pression du gaz d'une cartouche de Siphon (19) dans le cylindre d'inoculation, à l'encontre de la force d'un ressort de rappel (11) sous l'effet de la poussée d'un piston moteur (5) disposé dans l'unité d'actionnement (2), un dispositif de libération (13, 15, 29, 30) apte à être mis sous tension pour l'actionnement brutal d'une valve (18) d'actionnement par mise sous pression du piston moteur (5), un corps étalon (21) fixé sur la tige du piston d'inoculation (4) et une butée prévue sur l'unité d'actionnement (2) pour limiter la course d'avance du piston d'inoculation (4), caractérisé en ce que le corps étalon (21) est fixé entre la face soumise à la pression du piston moteur (5) et le piston d'inoculation (4) à l'extrémité de la tige de ce dernier et en ce que le piston moteur (5) poussant le corps étalon (21) pour déplacer le piston d'inoculation (4) coopère avec la butée.

0 133 471